# EUROPEAN PATENT SPECIFICATION

(11) **EP 0 383 429 B1**
(45) Date of publication and mention of the grant of the patent: **08.11.1995**
(21) Application number: 90300438.0
(22) Date of filing: 16.01.1990
(51) Int. Cl.: A61M 25/10, A61M 31/00, A61M 29/02

(54) **Catheter and method for locally applying medication to the wall of a blood vessel or other body lumen**
Katheter und Methode zur lokal angewandten Medikation der Wand eines Blutgefässes oder eines anderen Körperlumens
Cathéter et méthode pour médication appliquée localement sur la paroi d'un vaisseau sanguin ou d'une autre cavité corporelle

(30) Priority: 31.01.1989 US 304352
(43) Date of publication of application: 22.08.1990
(73) Proprietor: C.R. BARD, INC., Murray Hill New Jersey 07974 (US)
(72) Inventor: Wolinsky, Harvey, New York, NY 10128 (US); Barbere, Michael D., Dunstable, Massachusetts 01827 (US); King, Spencer L., Atlanta, Georgia 30307 (US)
(74) Representative: Woodward, John Calvin

(56) References cited:
- EP-A- 0 205 851
- EP-A- 0 284 672
- EP-A- 0 338 979
- US-A- 4 490 421

## Description

The invention relates to catheters and techniques for treating, with medicine, drugs or the like, a blood vessel or other body organ having a lumen through which the blood vessel or organ may be accessed.

Often, in the treatment of various diseases, it may be desirable to treat a body organ, blood vessel or the like with medicine or drugs in a high concentration. Although desirable, the practice often may not be achievable because in order to apply the potentially toxic medicament in a sufficiently high dose to be effective in the region to be treated, the body may become flooded with dangerously high levels of the medicament which could result in damage to other parts of the body or could even be life threatening. For example, it has been found experimentally in animals that very high' concentrations of heparin (or a particular component of heparin) when infused into the blood circulation tend to inhibit smooth muscle cell proliferation. See "Inhibition of Rat Arterial Smooth Muscle Cell Proliferation by Heparin" by John R. Guyton, et al., Circulation Research, Vol. 46, No. 5, May 1980, pp. 625-633; and "Vascular Smooth Muscle Proliferation Following Balloon Injury is Synergistically Inhibited by Low Molecular Weight Heparin and Hydrocortisone", John V. Gordon, et al., Circulation 76:IV-213, 1987. This may be of significance in connection with percutaneous transluminal angioplasty procedures by which an arterial stenosis is expanded to restore blood flow through an artery. Among the significant problems currently facing physicians practising angioplasty is that there is a relatively high rate of restenosis (of the order of 30%) after performing the initial angioplasty. It is believed that a significant contributing factor to restenosis may be smooth muscle cell proliferation of the artery wall. See "Intimal Proliferation of Smooth Muscle Cells as an Explanation for Recurrent Coronary Artery Stenosis After percutaneous Transluminal Coronary Angioplasty" by Garth E. Austin, et al., Journal American College of cardiology, Vol. 6, No. 2, August 1985, pp. .369-375. Thus, it may be advantageous to apply concentrated doses of heparin to an arterial wall that has been treated with angioplasty since effective doses of the heparin would be dangerous if introduced into the general circulation.

Although the desirability of applying high doses of medication to a local region of an artery or other body vessel has been recognized (see applicant's copending application Serial No. 042,569 filed April 21, 1987 and U.S. Patent 4,423,725 issued January 3, 1984 to Baran), there remains a need for an improved catheter for delivering such high concentrations without also flooding the patient's system with an unacceptably large concentration of the medication.

It is among the general objects of the invention to provide a method and apparatus for delivering high concentrations of medication to a localized area of a body vessel and to apply such medicine under pressure to cause penetration of the medicine into the wall of the body vessel, but without delivering large doses of the medication to the patient's overall system.

The invention relates to a catheter, for applying a liquid to a surface of the lumen of a body vessel, comprising an elongated flexible shaft, having a proximal end and a distal end with an inflation lumen extending from its proximal toward its distal end, and a balloon, having a plurality of perforations, mounted on the distal end of the shaft, the interior of the balloon being in communication with the inflation lumen. A catheter of this type is disclosed in EP-A-0 205 851 . The flow area defined by the perforations in the balloon, in catheters in accordance with the invention, is selected to provide a very low, weeping flow rate, typically of the order of a few cubic centimeters per minute through the balloon wall, the maximum flow rate depending on the particular medicine or drug being delivered.

Thus, in a first aspect, the invention provides a catheter of the aforementioned type characterised in that the balloon is flexible and substantially inelastic, the size and number of said perforations and the thickness of the balloon wall are selected such that a saline solution can flow through the perforations at a rate of 2-12 ml/minute when it is supplied to the interior of the balloon at a pressure of 0.2-0.5MPa (2-5 atmospheres), and the perforations define a flow area sufficiently small so as not to adversely restrict collapsing of the balloon, about the catheter shaft, under the influence of aspiration applied to the inflation lumen, the catheter being for effecting penetration of the liquid into the body vessel. In a second aspect, the invention provides such a catheter characterised in that the size and number of said perforations and the thickness of the balloon wall are selected such that a saline solution can flow through the perforations at a rate of up to 12 ml/minute when it is supplied to the interior of the balloon at a pressure of about 0.5MPa (5 atmospheres).

The catheter should be selected with respect to the diameter of the lumen in the body vessel to be treated so that the inflated diameter of the balloon is slightly greater than the lumen diameter. Thus, when the balloon is inflated within the lumen it will press firmly against the surface of the body lumen. Once the balloon has been inflated by the medication solution, continued pressure applied to the interior of the balloon will force the medication through the perforations and between the balloon and the luminal surface of the body vessel. The medication spreads between the balloon and the body lumen, forming a thin film, and the film is maintained under pressure by continually pressurizing the fluid in the catheter and the balloon. The continued pressure of the film forces the high-dose medication to penetrate the wall of tissue defining the body lumen while excess fluid bleeds off into the patient's system but at such a low volume that no adverse effect on other body organs or systems results. Such a film pressure may be maintained for up to several minutes to cause the medication to penetrate the tissue to the extent desired without introducing into the patient's system a large quantity of drugs.

Preferred embodiments of the invention are set out in claims 3-12 appended hereto.

The foregoing and other objects and advantages of the invention will be appreciated more fully from the following further description thereof, with reference to the accompanying drawings wherein:
FIG. 1 is an illustration of the balloon catheter used in the practice of the invention;
FIG. 2 is an enlarged longitudinal cross-sectional illustration of the catheter taken through the region of the balloon as seen along the line 2-2 of FIG. 1;
FIG. 3 is an illustration of the balloon laid flat showing the locations of holes as they may be formed by a laser;
FIG. 4 is a side view of the flattened balloon as seen from an end thereof;
FIG. 5 is a sectional illustration through the balloon illustrating the circumferential spacing of the rows of holes;
FIG. 6 is an illustration of the balloon in a deflated condition prior to insertion of the balloon into a patient's artery;
FIG. 7 is an enlarged illustration of the balloon portion of the catheter in an artery and in an inflated configuration; and
FIG. 8 is an enlarged cross-sectional illustration of the boundary region of the balloon, fluid film and artery.

### DESCRIPTION OF THE ILLUSTRATIVE EMBODIMENT

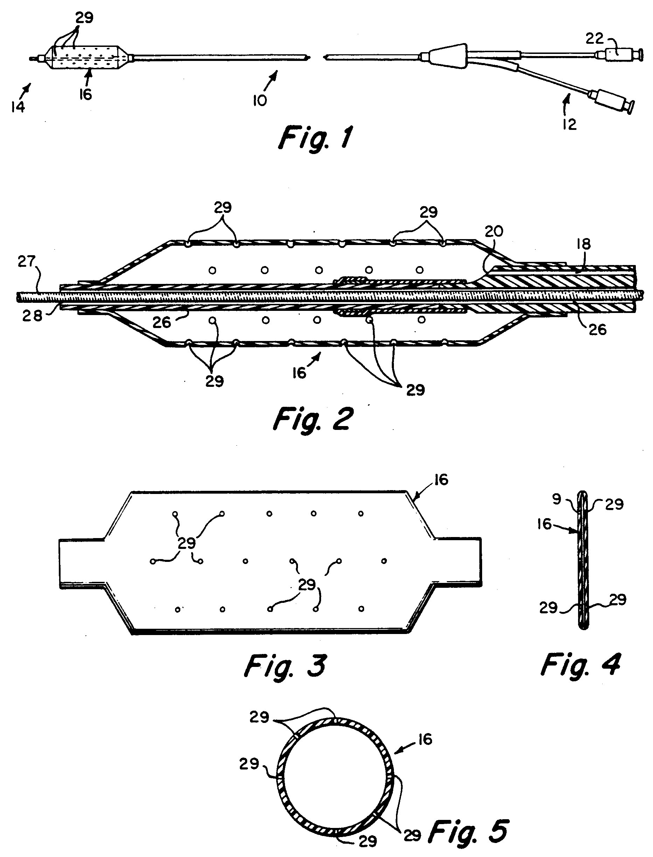

FIG. 1 illustrates the catheter used in the practice of the invention. The catheter includes an elongate flexible shaft 10 that may be formed in an extrusion process from an appropriate plastic material such as polyethylene. By way of example, when the catheter is intended to be used in the coronary arteries, the shaft may be of the order of 150 cm long and may have an outer diameter of 1.4 mm (.054"). The catheter has a proximal end 12 and a distal end 14. An inflatable and deflatable balloon, indicated generally at 16 is mounted on the distal end 14 of the catheter shaft 10. As shown in FIG. 2, the catheter shaft 10 includes an inflation lumen 18 that extends from the proximal end of the shaft and terminates in an opening 20 within the balloon 16. The proximal end 12 of the catheter shaft 10 carries a fitting 22 by which the inflation lumen 18 may be connected to a syringe (not shown) or other pressure fluid delivery device. The catheter shaft 10 also may be formed to include a guidewire lumen 26 that extends to and terminates in an outlet orifice 28 at the distal tip of the catheter shaft 10. The guidewire lumen 26 may be used to receive a guidewire 27 by which the catheter may be guided through a patient's vasculature to the site to be treated.

The balloon 16 may be formed from various polymeric materials and desirably has a thin, flexible, inelastic wall. A preferred material is polyethylene terephthalate and a balloon having a wall thickness of 0,025 mm (.001") or less may be fabricated as described in U.S. Patent 4,490,421 (Levy) or EP-A-0 279 411. By way of example, a catheter according to the invention adapted for use in the coronary arteries may have a balloon about 20 mm or more long and a wall thickness of 0.025 mm (.001") or less. It is contemplated that with a inelastic balloon several different sizes of (inflated) balloons may be required, depending on the application in which the catheter is to be used. For example, when used as an adjunct to angioplasty of the coronary arteries, balloons having diameters of 2.0 mm, 2.5 mm, 3.0 mm, 3.5 mm may be appropriate. In general, in connection with arterial angioplasty, it is contemplated that the balloon of the present invention should be selected to match the balloon used in the angioplasty procedure, the foregoing range being typical of the balloon sizes commonly employed in coronary angioplasty catheters.

The balloon 16 is provided with a plurality of minute holes 29 that are substantially regularly spaced about the balloon 16. For example, we have found that an array of about thirty holes, each about 25µm (microns) in diameter has been satisfactory. The holes may be formed by a laser beam from an excimer laser having wavelengths of 248 or 308 nm. These have been found to form clean edged holes in the balloon material. FIGS. 2 and 3 illustrate a satisfactory pattern of holes including six longitudinally extending rows having five holes 30 in each row. Some of the rows may be staggered longitudinally, with respect to each other as illustrated in FIG. 2. The holes are formed before the balloon is mounted on the catheter shaft. The balloon is laid flat as shown in FIG. 4 while a laser beam is used to drill the holes 29 in the desired pattern shown. In that pattern, the holes extend over a length of about 12 mm of the balloon and are located on 1.2 mm centers for a 3.0 mm diameter balloon.

The aggregate flow area defined by the holes 29 is selected so that under the general range of inflation pressures expected, the liquid flow through the holes will be very low, weeping in nature, and will not exceed a predetermined maximum flow rate in atmosphere. Although the foregoing configuration of holes is believed to be satisfactory for a wide range, and possibly most, if not all, medications or drugs to be delivered, it is possible that certain medications or drugs may have viscosity and flow characteristics as might require modification to the holes. The foregoing array of holes has been found to produce satisfactorily low flow rates for fluid medications having a viscosity and fluid characteristic similar to saline (such as heparin). In accordance with a preferred embodiment of the invention, the maximum flow rate is between 2 to 12 ml (cc) per minute under inflation pressures of 0.2 - 0.5MPa (2 to 5 atmospheres). Additionally, it is important that the holes 29 do not define a flow area so large as would impair the ability of the balloon to be collapsed when the inflation lumen is aspirated. The flow area should be not so large that there is a significant fluid loss to any side branches that the balloon may be inflated across. This is after all the original problem with double balloon catheters and solved by the instant invention. Collapse of the balloon is necessary in order to permit the balloon to be withdrawn from the patient's vasculature.

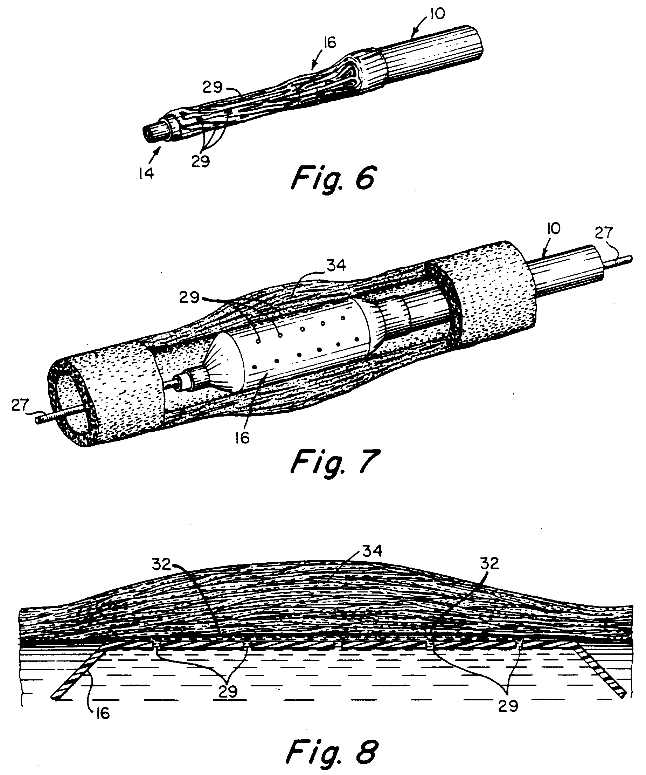

Use of the catheter and practice of the method may be illustrated in arterial angioplasty, such as percutaneous transluminal coronary angioplasty. Typically, the angioplasty procedure will have been performed by the physician according to any of a variety of techniques using various angioplasty catheters available. For purposes of illustration, it may be assumed that the angioplasty procedure will have been performed either by a balloon catheter, laser catheter or other angioplasty catheter to enlarge the stenosed region to a luminal diameter of 2.5 mm. Typically, the arterial wall will display a certain amount of "recoil" after the angioplasty so that the luminal diameter may be slightly smaller than 2.5 mm. In order thereafter to treat the arterial wall with concentrated heparin (or an isolated heparin fraction having antiproliferative effect), the angioplasty catheter is removed from the patient, typically permitting. the guidewire to remain in place. The catheter of the present invention will have been prepared, as by the physician's assistant, to fill the inflation lumen 18 and interior of the balloon 16 with the medication, taking care to purge the inflation and balloon system of air. The balloon then is wrapped about the catheter shaft to collapse the balloon to a low profile, as suggested by FIG. 6, by which it may be passed through the artery (or a guiding catheter leading to the coronary artery to be treated). The catheter then is advanced over the guidewire which guides the catheter to the site of the angioplasty. The catheter preferably is provided with one or more radiopaque marker bands by which the balloon position may be monitored under fluoroscopy to verify placement of the balloon in the region to be treated. Once the balloon is positioned within the site of the angioplasty, the syringe or other inflation device is operated to pressurize the catheter lumen and interior of the balloon to cause the balloon to inflate as suggested in FIG. 7. By proper selection of the balloon size, the balloon will inflate into close pressing contact with the inner surface of the arterial lumen, effecting intimate surface contact with the lumen. Pressure is applied continually at the inflation device (which may be fitted with a pressure gauge) to maintain a substantially constant pressure level as desired, the range of pressures anticipated being of the order of 0,2-0,5MPa(2 to 5 atmospheres). The pressure is sufficient to press the balloon firmly against the luminal surface of the artery but not so great as to preclude medication from weeping through the holes 29 in the balloon. As the medication weeps through the holes, it spreads into a generally cylindrical film 32 between the balloon 16 and artery wall 34. Because of the tendency of the balloon to intimately contact the artery wall, the film 32 will be maintained under pressure and will be forced into the arterial wall. Although there is some fluid pressure drop as the fluid weeps through the holes 29 and between the balloon and artery wall, the film pressure remains sufficiently high that the medication will progressively proliferate through the arterial tissue. The pressure and flow will be continued for a predetermined time, for example, one to two minutes, as is sufficient to achieve the desired penetration of medication into the arterial wall. Preferably, the holes are placed so that the end most of the holes 29 are spaced inwardly somewhat from the conical ends of the balloon thereby defining an annular portion at each end of the balloon which is unperforated and which may tend to have a partial sealing effect, tending to retard flow of the fluid from out between the balloon and the artery wall. The balloon then is deflated by aspirating through the inflation/deflation lumen to cause the balloon to collapse. The balloon catheter then is withdrawn from the patient. The flow area defined by the holes is sufficiently minute and the balloon wall is sufficiently flexible so that the balloon will collapse under aspiration.

By way of example, based on animal experiments conducted in accordance with the invention, the following penetrations of heparin through the tissue of an artery may be expected under the pressure levels for about one minutes.

| Gage Pressure at Proximal Part of Catheter | Estimated Average Pressure of Film in Contact with Artery | Depth of Penetration Into Wall of Artery in 1 Minute | Approximate Flow Rate of Unrestricted Catheter |
|---|---|---|---|
| 0.2MPa(2 bar) | 0-6.6KPa (0-50 mm Hg) | (100µm(microns) | 2 ml/min |
| 0.3MPa(3 bar) | 19.9-39.9KPa (150-300 mm Hg) | (200µm(microns) | 3 ml/min |
| 0.4MPa(4 bar) | 39.9-66.4KPa(300-500 mm Hg) | (300-400µm(microns) | 4 ml/min |
| 0.5MPa(5 bar) | 66.4-133KPa (500-1000 mm Hg) | (500-700µm(microns) | 5 ml/min |

Thus, we have described the invention by which highly concentrated medication may be applied to a surface of a body lumen, such as an artery, under sufficient pressure to cause the medication to penetrate into the tissue without introducing excessively high volumes of the medication into the patient's system. It should be understood, however, that although the invention has been described principally in connection with post-angioplasty treatment of an artery with heparin or an antiproliferative fraction of heparin, the invention may be practised in any instance where it is desired to apply a high concentration of medication to a local vessel or organ having a lumen accessible by a catheter. Thus the invention may be used to deliver chemotherapeutic drugs in the treatment of cancer patients where it is desired to apply concentrated medication or chemotherapeutic agents to the diseased organ. The catheter may be passed into a lumen in the organ or may even be inserted into a lumen formed in the organ or tumor for the express purpose of receiving the catheter. Therefore, it should be understood that other embodiments and modifications of the invention may be apparent to those skilled in the art.

## Claims

1. A catheter, for applying a liquid to a surface of the lumen of a body vessel, comprising an elongated flexible shaft (10), having a proximal end and a distal end with an inflation lumen (18) extending from its proximal toward its distal end, and a balloon (16), having a plurality of perforations (29), mounted on the distal end of the shaft, the interior of the balloon being in communication with the inflation lumen, characterised in that the balloon is flexible and substantially inelastic, the size and number of said perforations (29) and the thickness of the balloon wall are selected such that a saline solution can flow through the perforations at a rate of 2-12 ml/minute when it is supplied to the interior of the balloon (16) at a pressure of 0.2-0.5MPa (2-5 atmospheres), and the perforations (29) define a flow area sufficiently small so as not to adversely restrict collapsing of the balloon (16), about the catheter shaft (10), under the influence of aspiration applied to the inflation lumen, the catheter being for effecting penetration of the liquid into the body vessel.

2. A catheter, for applying a liquid to a surface of the lumen of a body vessel, comprising an elongated flexible shaft (10), having a proximal end and a distal end with an inflation lumen (18) extending from its proximal toward its distal end, and a balloon (16), having a plurality of perforations (29), mounted on the distal end of the shaft, the interior of the balloon being in communication with the inflation lumen, characterised in that the balloon is flexible and substantially inelastic, the size and number of said perforations (29) and the thickness of the balloon wall are selected such that a saline solution can flow through the perforations at a rate of up to 12 ml/minute when it is supplied to the interior of the balloon (16) at a pressure of about 0.5MPa (5 atmospheres), and the perforations (29) define a flow area sufficiently small so as not to adversely restrict collapsing of the balloon (16), about the catheter shaft (10), under the influence of aspiration applied to the inflation lumen, the catheter being for effecting penetration of the liquid into the body vessel.

3. A catheter as claimed in claim 1 or claim 2, characterised in that the flow rate through the perforations is 2-5ml/minute.

4. A catheter as claimed in claim 1 or claim 2, characterised in that the flow rate through the perforations is about 1 ml/minute per 0.1 MPa (1 atmosphere) of pressure applied to the saline supplied to the balloon.

5. A catheter as claimed in any of the preceding claims, characterised in that the balloon (16) has an inflated diameter of 2-3.5mm.

6. A catheter as claimed in any of the preceding claims characterised in that the balloon (16) is formed from polyethylene terephthalate.

7. A catheter as claimed in any of the preceding claims characterised in that the balloon (16) is provided with about thirty (29) substantially regularly spaced perforations (29), each having a diameter of about 25µm (microns), and the thickness of the balloon (16) wall is 0.025mm (.001") or less.

8. A catheter as claimed in claim 7 characterised in that the catheter is dimensioned and adapted to be percutaneously inserted and advanced into the coronary arteries.

9. A catheter as claimed in any preceding claim characterised in that the balloon (16) includes a cylindrical perforated surface having an annular portion at each end that is free of perforations.

10. A catheter as claimed in any preceding claim characterised in that the pressure is 0.4MPa (4 atmospheres).

11. A catheter as claimed in any of claims 1-9 characterised in that the pressure is 0.3MPa (3 atmospheres).

12. A catheter as claimed in any of claims 1-9 characterised in that the pressure is 0.2MPa (2 atmospheres).

## Patentansprüche

1. Katheter zum Aufbringen einer Flüssigkeit auf eine Oberfläche einer Kavität eines Körpergefäßes, mit einem verlängerten, flexiblen Schaft (10) mit einem nahen und einem fernen Ende mit einer aufblasbaren Kavität (18), die sich von seinem nahen zu seinem fernen Ende erstreckt, sowie mit einem Ballon (16) mit einer Mehrzahl von Perforationen (29), der an dem entfernten Ende des Schaftes angeordnet ist und dessen Innenraum in Verbindung mit der aufblasbaren Kavität steht, dadurch gekennzeichnet, daß der Ballon flexibel und im wesentlichen unelastisch ist, daß die Größe und Anzahl der Perforationen (29) und die Dicke der Ballonwand so gewählt sind, daß eine Salzlösung mit einer Geschwindigkeit von 2 bis 12 ml pro Minute durch die Perforationen fließen kann, wenn diese dem Inneren des Ballons (16) mit einem Druck von 0,2 bis 0,5 MPa (2 bis 5 Atmosphären) zugeführt wird, und daß die Perforationen (29) einen Fließbereich bilden, der genügend klein ist, um nicht umgekehrt ein Zusammenfallen des Ballons (16) um den Schaft (10) des Katheters zu behindern, wobei der Katheter dazu vorgesehen ist, unter dem Einfluß einer in die aufblasbare Kavität eingebrachten Aspiration ein Eindringen der Flüssigkeit in die Körperkavität zu bewirken.

2. Katheter zum Aufbringen einer Flüssigkeit auf eine Oberfläche einer Kavität eines Körpergefäßes, mit einem verlängerten, flexiblen Schaft (10) mit einem nahen und einem fernen Ende mit einer aufblasbaren Kavität (18), die sich von seinem nahen zu seinem fernen Ende erstreckt, sowie mit einem Ballon (16) mit einer Mehrzahl von Perforationen (29), der an dem entfernten Ende des Schaftes angeordnet ist und dessen Innenraum in Verbindung mit der aufblasbaren Kavität steht, dadurch gekennzeichnet, daß der Ballon flexibel und im wesentlichen unelastisch ist, daß die Größe und Anzahl der Perforationen (29) und die Dicke der Ballonwand so gewählt sind, daß eine Salzlösung mit einer Geschwindigkeit von bis zu 12 ml pro Minute durch die Perforationen fließen kann, wenn diese dem Inneren des Ballons (16) mit einem Druck von etwa 0,5 MPa (5 Atmosphären) zugeführt wird, und daß die Perforationen (29) einen Fließbereich bilden, der genügend klein ist, um nicht umgekehrt ein zusammenfallen des Ballons (16) um den Schaft (10) des Katheters zu behindern, wobei der Katheter dazu vorgesehen ist, unter dem Einfluß einer in die aufblasbare Kavität eingebrachten Aspiration ein Eindringen der Flüssigkeit in die Körperkavität zu bewirken.

3. Katheter nach Anspruch 1 oder 2, dadurch gekennzeichnet, daß die Fließrate durch die Perforationen 2 bis 5 ml pro Minute beträgt.

4. Katheter nach Anspruch 1 oder 2, dadurch gekennzeichnet, daß die Fließrate durch die Perforationen etwa 1 ml pro Minute und pro 0,1 MPa (1 Atmosphären) Druck auf die dem Ballon zugeführte Salzlösung beträgt.

5. Katheter nach mindestens einem der vorhergehenden Ansprüche, dadurch gekennzeichnet, daß der Ballon (16) in aufgeblasenem Zustand einen Durchmesser von 2 bis 3,5 mm aufweist.

6. Katheter nach mindestens einem der vorhergehenden Ansprüche, dadurch gekennzeichnet, daß der Ballon (16) aus Polyethylen-Terephthalat gebildet ist.

7. Katheter nach mindestens einem der vorhergehenden Ansprüche, dadurch gekennzeichnet, daß der Ballon (16) mit etwa 30 im wesentlichen regelmäßig beabstandeten Perforationen (29) mit einem Durchmesser von jeweils etwa 25 µm (Mikrons) versehen ist und die Dicke der Ballonwand 0,025 mm (0,001") oder weniger beträgt.

8. Katheter nach Anspruch 7, dadurch gekennzeichnet, daß der Katheter so bemessen und angepaßt ist, daß er perkutan eingeführt und in den koronaren Arterien weitergeführt werden kann.

9. Katheter nach mindestens einem der vorhergehenden Ansprüche, dadurch gekennzeichnet, daß der Ballon (16) eine zylindrische, perforierte Oberfläche mit einem ringförmigen Bereich an jedem, von Perforationen freien Ende aufweist.

10. Katheter nach mindestens einem der vorhergehenden Ansprüche, dadurch gekennzeichnet, daß der Druck 0,4 MPa (4 Atmosphären) beträgt.

11. Katheter nach mindestens einem der Ansprüche 1 bis 9, dadurch gekennzeichnet, daß der Druck 0,3 MPa (3 Atmosphären) beträgt.

12. Katheter nach mindestens einem der Ansprüche 1 bis 9, dadurch gekennzeichnet, daß der Druck 0,2 MPa (2 Atmosphären) beträgt.

## Revendications

1. Cathéter destiné à l'application d'un liquide à une surface de la lumière d'un vaisseau corporel, comprenant un arbre souple allongé (10) ayant une extrémité proximale et une extrémité distale, avec une lumière de gonflage (18) s'étendant depuis son extrémité proximale vers son extrémité distale, et un ballon (16) ayant une pluralité de perforations (29) monté sur l'extrémité distale de l'arbre, l'intérieur du ballon étant en communication avec la lumière de gonflage,
caractérisé en ce que
le ballon est souple et pratiquement non élastique, la dimension et le nombre desdites perforations (29) et l'épaisseur de la paroi du ballon sont sélectionnés de manière telle qu'une solution saline puisse s'écouler à travers les perforations à un débit de 2 - 12 ml/ minute quand elle est amenée à l'intérieur du ballon (16) sous une pression de 0,2 - 0,5 MPa (2 - 5 atmosphères) et les perforations (29) définissent une région d'écoulement suffisamment petite pour ne pas restreindre de façon gênante l'aplatissement du ballon (16) autour de l'arbre (10) sous l'influence d'une aspiration appliquée à la lumière de gonflage, le cathéter étant prévu pour effectuer la pénétration du liquide à l'intérieur du vaisseau corporel.

2. Cathéter destiné à l'application d'un liquide à une surface de la lumière d'un vaisseau corporel, comprenant un arbre souple allongé (10) ayant une extrémité proximale et une extrémité distale, avec une lumière de gonflage (18) s'étendant depuis son extrémité proximale vers son extrémité distale, et un ballon (16) ayant une pluralité de perforations (29) monté sur l'extrémité distale de l'arbre, l'intérieur du ballon étant en communication avec la lumière de gonflage,
caractérisé en ce que
le ballon est souple et pratiquement non élastique, la dimension et le nombre desdites perforations (29) et l'épaisseur ou la paroi du ballon sont sélectionnés de manière telle qu'une solution saline puisse s'écouler à travers les perforations à un débit allant jusqu'à 12 ml/ minute quand elle est amenée à l'intérieur du ballon (16) sous une pression d'environ 0,5 MPa (5 atmosphères) et les perforations (29) définissent une région d'écoulement suffisamment petite pour ne pas restreindre de façon gênante l'aplatissement du ballon (16) autour de l'arbre du cathéter (10) sous l'influence d'une aspiration appliquée à la lumière de gonflage, le cathéter étant prévu pour effectuer la pénétration du liquide dans le vaisseau corporel.

3. Cathéter selon la revendication 1 ou la revendication 2, caractérisé en ce que le débit à travers les perforations est de 2 - 5 ml/ minute.

4. Cathéter selon la revendication 1 ou la revendication 2, caractérise en ce que le débit à travers les perforations est d'environ 1 ml / minute pour 0,1 MPa (1 atmosphère) de pression appliquée à la solution saine amenée au ballon.

5. Cathéter selon l'une quelconque des revendications précédentes, caractérisé en ce que le ballon (16) a un diamètre de 2 - 3,5 mm à l'état gonflé.

6. Cathéter selon l'une quelconque des revendications précédentes, caractérisé en ce que le ballon (16) est réalisé en téréphtalate de polyéthylène.

7. Cathéter selon l'une quelconque des revendications précédentes, caractérisé en ce que le ballon (16) comporte environ trente perforations (29) régulièrement espacées, ayant chacune un diamètre d'environ 25 µm (microns) et l'épaisseur de la paroi du ballon (16) est de 0,025 mm au moins.

8. Cathéter selon la revendication 7, caractérisé en ce que le ballon (16) est dimensionné et adapté de manière à être inséré et avancé par voie percutanée dans les artères coronaires.

9. Cathéter selon l'une des revendications précédentes, caractérisé en ce que le ballon (16) comporte une surface perforée cylindrique ayant à chaque extrémité une portion annulaire exempte de perforations.

10. Cathéter selon l'une quelconque des revendications précédentes, caractérisé en ce que la pression est de 0,4 MPa (4 atmosphères).

11. Cathéter selon l'une quelconque des revendications 1 - 9, caractérisé en ce que la pression est de 0,3 MPa (3 atmosphères).

12. Cathéter selon l'une quelconque des revendications 1 - 9, caractérisé en ce que la pression est de 0,2 MPa (2 atmosphères).
